# EUROPEAN PATENT APPLICATION

(11) **EP 0 671 413 A1**
(43) Date of publication of application: **13.09.1995**
(21) Application number: 95810088.5
(22) Date of filing: 10.02.1995
(51) Int. Cl.: C07K 14/655

(54) **Somatostatin analogs for preventing NSAID-induced ulcers**

(30) Priority: 14.02.1994 GB 9402767
(71) Applicant: SANDOZ LTD, CH-4002 Basel (CH); SANDOZ-PATENT-GMBH, D-79539 Lörrach (DE); SANDOZ ERFINDUNGEN VERWALTUNGSGESELLSCHAFT M.B.H., A-1235 Vienna (AT)
(72) Inventor: Buchheit, Karl-Heinz, D-79539 Lörrach (DE); Engel, Günter, D-79576 Weil (DE); Gamse, Rainer, CH-4054 Basle (CH)

(57) **Abstract**

Use of a somatostatin analogue of formula I in the manufacture of a pharmaceutical composition for use in preventing or treating NSAID induced gastro-intestinal lesions or ulcers.

## Description

The present invention relates to a new use, in particular a new use for the compound group comprising somatostatin analogues and derivatives, in free form or in pharmaceutically acceptable salt or complex form.

Suitable somatostatin analogues and derivatives include e.g. compounds of formula I 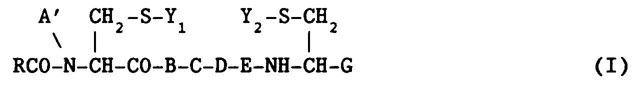
wherein RCO- is
i) an L- or D-phenylalanine residue optionally ring-substituted by halogen, NO₂, NH₂, OH, C₁₋₃alkyl and/or C₁₋₃alkoxy;
ii) the residue of a natural or a synthetic α-amino-acid other than defined under i) above, or of a corresponding D-amino acid, or
iii) a dipeptide residue in which the individual amino acid residues are the same or different and are selected from those defined under i) and/or ii) above,

the α-amino group of amino acid residues i) and ii) and the N-terminal amino group of dipeptide residues iii) being optionally mono- or di-C₁₋₁₂alkylated or substituted by C₁₋₈alkanoyl;
A' is hydrogen or C₁₋₃alkyl,
Y₁ and Y₂ represent together a direct bond or each of Y₁ and Y₂ is independently hydrogen or a radical of formulae (1) to (5)

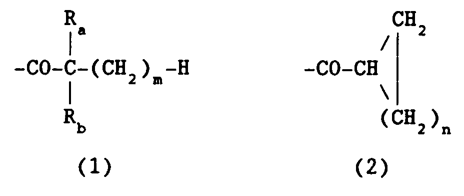

-CO-NHR_{c} (3)

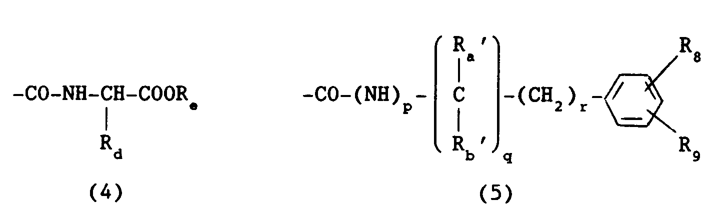

wherein
- Rₐ: is methyl or ethyl
- R_{b}: is hydrogen, methyl or ethyl
- m: is a whole number from 1 to 4
- n: is a whole number from 1 to 5
- R_{c}: is (C₁₋₆)alkyl
- R_{d}: represents the substituent attached to the α-carbon atom of a natural or synthetic α-amino acid (including hydrogen)
- Rₑ: is (C₁₋₅)alkyl
- Rₐ' and R_{b}': are independently hydrogen, methyl or ethyl,
- R₈ and R₉: are independently hydrogen, halogen, (C₁₋₃)alkyl or (C₁₋₃)alkoxy,
- p: is 0 or 1,
- q: is 0 or 1, and
- r: is 0, 1 or 2,
- B: is -Phe- optionally ring-substituted by halogen, NO₂, NH₂, OH, C₁₋₃alkyl and /or C₁₋₃alkoxy (including pentafluoroalanine), or naphthylalanine
- C: is (L)-Trp- or (D)-Trp- optionally α-N-methylated and optionally benzene-ring-substituted by halogen, NO₂, NH₂, OH, C₁₋₃alkyl and/or C₁₋₃alkoxy,
- D: is Lys, Lys in which the side chain contains O or S in β-position, optionally α-N-methylated, or a 4-aminocyclohexylAla or 4-aminocyclohexylGly residue
- E: is Thr, Ser, Val, Tyr, Ile or an aminobutyric or aminoisobutyric acid residue
- G: is a group of formula

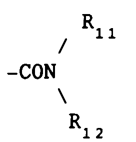

wherein
- R₁₁: is hydrogen, C₁₋₃alkyl, phenyl or C₇₋₁₀phenyl-alkyl,
- R₁₂: is hydrogen, C₁₋₃alkyl or a group of formula -CH(R₁₃)-X₁,
- R₁₃: is CH₂OH, -(CH₂)₂-OH, -(CH₂)₃-OH, or -CH(CH₃)OH or represents the substituent attached to the α-carbon atom of a natural or synthetic α-amino acid (including hydrogen),
- X₁: is a group of formula -COOR₇, -CH₂OR₁₀ or 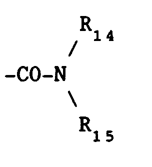
- R₇: is hydrogen or C₁₋₃alkyl,
- R₁₀: is hydrogen or the residue of a physiologically acceptable, physiologically hydrolysable ester,
- R₁₄: is hydrogen or C₁₋₃alkyl and
- R₁₅: is hydrogen, C₁₋₃alkyl, phenyl or C₇₋₁₀phenylalkyl, and
- R₁₆: is hydrogen or hydroxy,

with the proviso that
when R₁₂ is -CH(R₁₃)-X₁ then R₁₁ is hydrogen or methyl,
wherein the residues B, D and E have the L-configuration, and the residues in the 2- and 7-position and any residues Y₁ 4) and Y₂ 4) each independently have the (L)- or (D)- configuration,
in free form or in pharmaceutically acceptable salt or complex form.

Halogen is preferably fluorine, chlorine or iodine, more preferably fluorine.

In formula I, the following significances are preferred either individually or in any combination or sub-combination:
1.1. When RCO- has the meaning i) this is preferably L- or D-Phe or -Tyr. More preferably RCO- is L- or D-Phe.
1.2. When RCO- has the meaning ii) or iii) the defined residue is preferably lipophilic. Preferred residues ii) are α-amino acid residues having a hydrocarbon side chain, e.g. alkyl with 3, preferably 4, or more C atoms, e.g. up to 7 C-atoms, or a naphthyl-methyl or heteroaryl side chain, e.g. pyridyl-methyl or indol-3-yl-methyl, said residues having the L- or D-configuration. Preferred residues iii) are dipeptide residues in which the individual amino acid residues are the same or different and are selected from those defined above.
1.3. Most preferably RCO- has the meaning as indicated in 1.1 above.
2. B is B', where B' is Phe or Tyr.
3. C is C', where C' is (D)Trp.
4. E is E', where E' is Ser, Val or Thr, especially Val or Thr, particularly Thr.
5. G is G', where G' is a group of formula 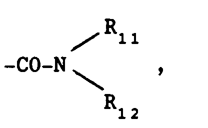 especially a group of formula 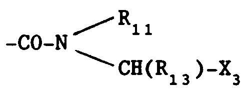 (in which case R₁₁=H or CH₃). In the latter case the moiety -CH(R₁₃)-X₃ preferably has the L-configuration.
6.1. R₁₁ is preferably hydrogen.
6.2. As the substituent attached to the α-carbon atom of a natural or non natural amino acid (i.e. of formula H₂N-CH(R₁₃)-COOH), R₁₃ is preferably -CH₂OH, -CH(CH₃)-OH, -(CH₂)₂-OH, -(CH₂)₃-OH, isobutyl, butyl, naphthyl-methyl or indol-3-yl-methyl. It is especially -CH₂OH or -CH(CH₃)OH.
6.3. X₃ is preferably a group of formula 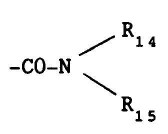 or -CH₂-OR₁₀, especially of formula -CH₂-OR₁₀ and R₁₀ is preferably hydrogen or as an ester residue formyl, C₂₋₁₂ alkylcarbonyl, C₈₋₁₂ phenylalkylcarbonyl or benzoyl. Most preferably R₁₀ is hydrogen. Preferably each of R₁₄ and R₁₅ is H.
7. Preferably Y₁ and Y₂ together represent a direct bond or each is H. Most preferably Y₁ and Y₂ together represent a direct bond.

Individual compounds of formula I suitable for use in accordance with the present invention are the following somatostatin analogues:

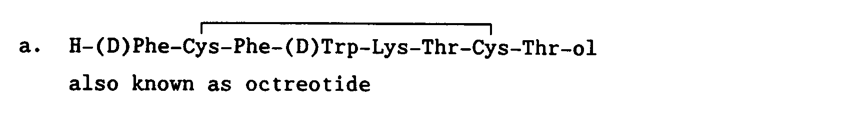

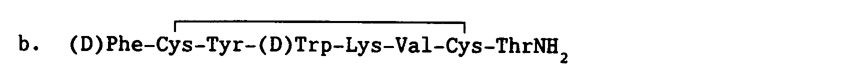

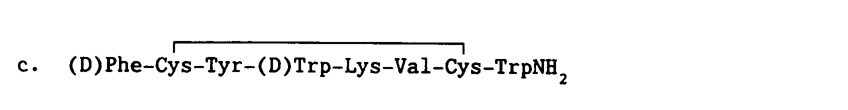

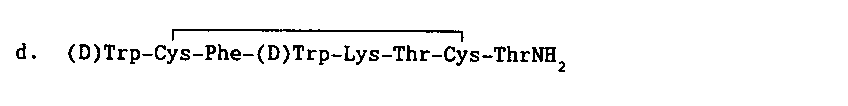

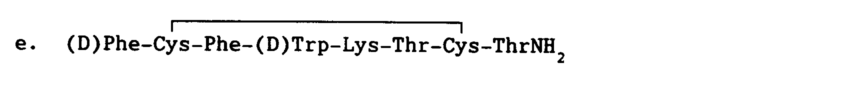

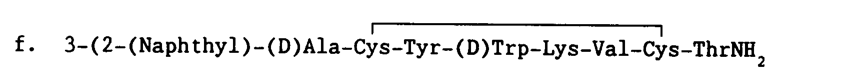

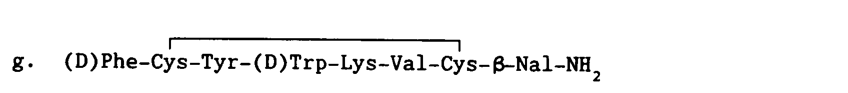

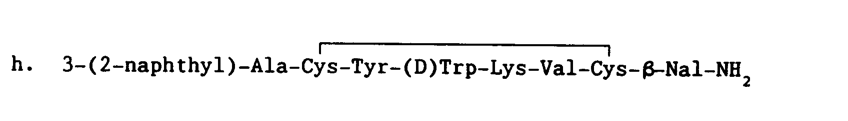

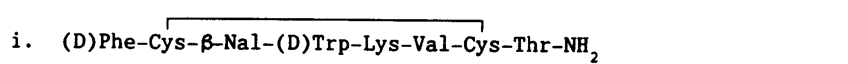

Compounds of formula I may exist e.g. in free form, salt form or in the form of complexes thereof. Acid addition salts may be formed with e.g. organic acids, polymeric acids and inorganic acids. Such acid addition salt forms include e.g. the hydrochlorides and acetates. Complexes are e.g. formed from compounds of the invention on addition of inorganic substances, e.g. inorganic salts or hydroxides such as Ca- and Zn-salts, and/or an addition of polymeric organic substances.

Compounds of formula I have, on the basis of observed activity, e.g.inhibition of GH secretion in rats and/or inhibition of pancreatic and gastric secretion in rats, e.g. as described in EP-B-29579 been found to be useful e.g. in the treatment of acromegaly, complications of diabetes mellitus or gastro-intestinal disorders.

In accordance with the present invention it has now surprisingly been found that compounds of formula I are particularly indicated for use as cytoprotective agents in the prevention or treatment of NSAID (Non Steroidal Antiinflammatory/Analgesic Drugs) induced gastrointestinal lesions and ulcers when used at a very low dose where they substantially do not inhibit gastric acid secretion.

In accordance with the particular findings of the present invention, there is provided in a first aspect:
I.1. A method for preventing or treating NSAID induced gastro-intestinal lesions or ulcers in a subject, which method comprises administering to said subject a compound of formula I or a pharmaceutically acceptable salt or complex thereof, in a dose at which the compounds of formula I substantially do not inhibit gastric acid secretion. Such a dose may be from 0.01 to 10 µg per day when administered parenterally, and from 10 µg to 1 mg per day, preferably 10 µg to 500 µg per day when administered orally.
   Compounds of formula I are particularly useful in the preventive or curative treatment of those patients who are exposed to additional gastro-irritants such as alcohol and cigarette smoke.
   In a yet further or alternative embodiment the invention provides:
I.2. A method for significantly reducing or suppressing the gastro-intestinal lesions and ulcers associated with a NSAID therapy in a subject, in need of such NSAID therapy comprising administering to said subject a therapeutically effective amount of a NSAID and a compound of formula I or a pharmaceutically acceptable salt or complex thereof, in a dose at which the compounds of formula I substantially do not inhibit gastric acid secretion. Preferably the compound of formula I is administered parenterally in a dose of from 0.01 to 10 µg per day, or orally in a dose of from 10 µg to 1 mg per day, preferably 10 to 500 µg/day. As alternatives to the above, the present invention also provides:
II. A compound of formula I or a pharmaceutically acceptable salt thereof for use in any method as defined under I.1 and I.2 above; or
III. Use of a compound of formula I or a pharmaceutically acceptable salt thereof in the manufacture of a pharmaceutical composition for use in the methods as defined above; or
   Use of a compound of formula I or a pharmaceutically acceptable salt thereof in the manufacture of a pharmaceutical composition for use in the methods as defined above, the treatment additionally comprising administration of at least one non-steroidal anti-inflammatory/analgesic drug (NSAID); or
IV. A composition for use in the prevention or treatment of conditions as hereinabove specified, in particular for use in the methods as defined above comprising a compound of formula I or a pharmaceutically acceptable salt or complex thereof together with one or more pharmaceutically acceptable diluents or carriers therefor: or
V. A therapeutic combination for use in reducing or suppressing conditions as hereinbefore specified, said combination including a pharmaceutical composition comprising a compound of formula I or a pharmaceutically acceptable salt or complex thereof and further including at least one pharmaceutical composition comprising a non-steroidal anti-inflammatory/analgesic drug (NSAID).

Utility of the compounds of formula I as cytoprotective agents in the treatment of diseases and conditions as hereinabove specified, may be demonstrated in animal models, for example in accordance with the method hereinafter described.

### A. Inhibition of Indomethacin-induced gastric lesions

Male rats (250 - 300 g) are fasted overnight. Throughout the experiments food and water are withheld. Test compounds are dissolved and diluted in saline and administered s.c. or orally. Indomethacin (10 mg/kg) is given orally 30 min later and the animals are killed after 4 hours. The stomach is cut open along the greater curvature and pinned flat on a cork covered with a white paper. (Under the conditions used, the stomachs of all animals are empty of remnants of food). The number and size of lesions are then determined. Six rats are used per group. Statistical analysis is done using the t-test.

On s.c. administration of a compound of formula I, e.g. octreotide, as test compound at a dosage of from ca. 0.01 to 10 ng/kg substantial inhibition of gastric lesions induced by Indomethacin is observed compared with results for control groups receiving placebo in lieu of test compound.

Compounds of formula I have a cytoprotective effect on the gastric mucosa against NSAID induced lesions and ulcers when administered in the nanogram range per kg body weight which is independent from the inhibition of gastric acid secretion.

On comparison compounds of formula I inhibit the gastric acid secretion at a considerably higher dose, for example in doses of from 0.3 to 300 µg/kg s.c. in rats.

The utility of the compounds of formula I is also confirmed in clinical trials, e.g. in patients with NSAID induced ulcers.

### B. Clinical Trial

I. After initial endoscopy to ensure mucosal integrity, 20 healthy adult volunteers (M:11, F:9, mean age 24 years) receive 50 mg Indomethacin po tid concomitantly with either an identical placebo (n = 5), or 0.01 µg (n = 5), 0.1 µg (n = 5) or 1.0 µg (n = 5) of a compound of formula I, e.g. octreotide, subcutaneously for 3 days. Both the subjects and the investigator are blinded with regard to the dose of compound of formula I. Endoscopy is then repeated and the extent of injury assessed. Observed injury is quantified using the following rating scale: normal (erythema included) = 0, ≦ 5 erosions = 1, 6-15 erosions = 2, > 15 erosions and/or spontaneous bleeding = 3, ulcer (≦ 4 mm + depth) = 4. For statistical trend analysis, a one-sided linear-by-linear associated test for ordered alternatives is used. P-values are calculated using the StatXact programme (Cytel Corp.). In this test, low doses of a compound of formula I co-administered with Indomethacin demonstrate a significant reduction in NSAID induced injury.
   When octreotide is used, the linear association test confirms a negative correlation between the octreotide dose and injury grade (p < 0.03 for gastric, p < 0.01 for duodenum).
II. Sixty patients either male or female with one or two endoscopically proven NSAID induced ulcers in the duodenal bulb are submitted to a double blind randomised study.
   The compound of formula I to be tested is administered s.c. Oesophagogastroduodenoscopy is performed on admission, at two weeks and at four weeks. Routine hematology and biochemistry, urinalysis and hormonal assays, including serum gastrin measurements, are also performed on entering the clinical trial, on the fourteenth (± 3 days), on the twenty eighth day (± 3 days) and at a follow visit 7 - 10 days later. Symptoms are assessed both by the investigator at the patient visits and by the patients by means of a diary card.
   During the course of the study, subjects receive a compound of formula I, e.g. octreotide at dosages of from 0.01 µg to 10 µg s.c. daily.
   Ulcer healing or prevention is assessed by endoscopy; healing is defined as reepithelialisation of the mucosa. Subjects receiving therapy in accordance with the invention exhibit significantly improved conditions.

Compounds of formula I used according to the invention have a beneficial effect on healing of NSAID induced gastro-intestinal lesions and ulcers.

Compounds of formula I may be administered orally or parenterally, e.g. s.c., preferably parenterally. Unit oral dosage forms suitably comprise from about 2.5 to 500 µg, preferably 2.5 to 250 µg, together with a pharmaceutical acceptable diluent or carrier therefor.

Pharmaceutical compositions for use in the method of the invention may be made in conventional manner. They may comprise for example a compound of formula I, in pharmaceutically acceptable form, e.g. salt form, in association with a pharmaceutical carrier or diluent.

As indicated above compounds of formula I can also be administered concomitantly with a NSAID to prevent gastro-intestinal intolerance of the latter. NSAID are widely administered orally in the treatment of inflammation e.g. acute or chronic, for example rheumatoid arthritis, osteoarthritis, and mild to moderate pain. Within this class, the compounds vary widely in their chemical structure and in their biological profiles as analgesics, anti-inflammatory agents and anti-pyretic agents. See for example Goodman and Gilman's "The Pharmacological Basis of Therapeutics", 8th Edition 1990 (Pergamon Press), the contents thereof relating to NSAIDs and the doses to be administered being incorporated herein by reference. Examples are e.g. diclofenac, indomethacin, diflunisal, zomepirac, ibuprofen, feneprofen, naproxen, piroxicam, flurbiprofen, sulindac, mefenamic acid etc. NSAID may be used in any amount known to be an effective analgesic or anti-inflammatory amount.

The compounds of formula I may be administered simultaneously with or before the selected NSAID. Where required, the selected NSAID may even be administered at a higher dose in order to achieve the desired effect, e.g. alleviate pain suffering more rapidly, due to the protective effect provided by the compounds of formula I.

The invention also provides kits or packages combining the NSAID pharmaceutical composition with a pharmaceutical composition comprising a compound of formula I. The kits or packages may also contain instructions to use the pharmaceutical compositions in accordance with the present invention.

Compounds of formula I are well tolerated at dosages required for use in accordance with the present invention. For example with an i.p. injection of octreotide at 100 mg/kg animal body no increase in GOT or any lethality is observed.

Pharmaceutically acceptable salt forms exhibit the same or similar levels of tolerability/activity as the compounds in free form.

Examples of an injectable composition comprising a compound of formula I, e.g. octreotide, is as follows:

| Vial | Concentration per ml |
|---|---|
| Octreotide | 0.01 mg |
| Mannitol | 45.0 mg |
| Lactic acid (88 %) | 3.4 mg |
| Sodium hydrogenocarbonate | to pH 4.2 |
| Water (inject. grade) | to 1 ml |
| Carbon dioxide | q.s. |

## Claims

1. Use of a compound of formula I 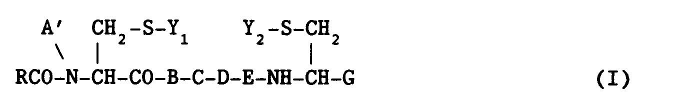 wherein RCO- is
i) an L- or D-phenylalanine residue optionally ring-substituted by halogen, NO₂, NH₂, OH, C₁₋₃alkyl and/or C₁₋₃alkoxy;
ii) the residue of a natural or a synthetic α-amino-acid other than defined under i) above, or of a corresponding D-amino acid, or
iii) a dipeptide residue in which the individual amino acid residues are the same or different and are selected from those defined under i) and/or ii) above,
the α-amino group of amino acid residues i) and ii) and the N-terminal amino group of dipeptide residues iii) being optionally mono- or di-C₁₋₁₂alkylated or substituted by C₁₋₈alkanoyl;
A' is hydrogen or C₁₋₃alkyl,
Y₁ and Y₂ represent together a direct bond or each of Y₁ and Y₂ is independently hydrogen or a radical of formulae (1) to (5) 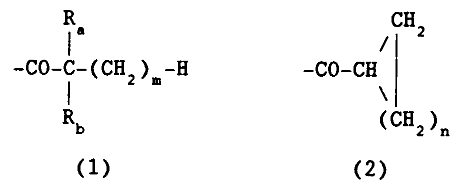
-CO-NHR_{c} (3) 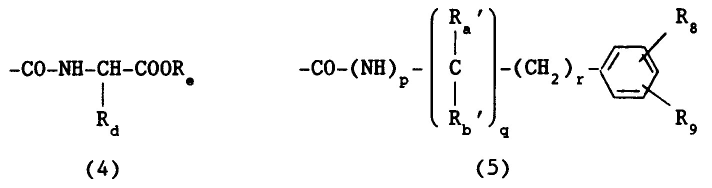
wherein
Rₐ is methyl or ethyl
R_{b} is hydrogen, methyl or ethyl
m is a whole number from 1 to 4
n is a whole number from 1 to 5
R_{c} is (C₁₋₆)alkyl
R_{d} represents the substituent attached to the α-carbon atom of a natural or synthetic α-amino acid (including hydrogen)
Rₑ is (C₁₋₅)alkyl
Rₐ' and R_{b}' are independently hydrogen, methyl or ethyl,
R₈ and R₉ are independently hydrogen, halogen, (C₁₋₃)alkyl or (C₁₋₃)alkoxy,
p is 0 or 1,
q is 0 or 1, and
r is 0, 1 or 2,
B is -Phe- optionally ring-substituted by halogen, NO₂, NH₂, OH, C₁₋₃alkyl and /or C₁₋₃alkoxy (including pentafluoroalanine), or naphthylalanine
C is (L)-Trp- or (D)-Trp- optionally α-N-methylated and optionally benzene-ring-substituted by halogen, NO₂, NH₂, OH, C₁₋₃alkyl and/or C₁₋₃alkoxy,
D is Lys, Lys in which the side chain contains O or S in β-position, optionally α-N-methylated, or a 4-aminocyclohexylAla or 4-aminocyclohexylGly residue
E is Thr, Ser, Val, Tyr, Ile or an aminobutyric or aminoisobutyric acid residue
G is a group of formula 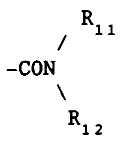
wherein
R₁₁ is hydrogen, C₁₋₃alkyl, phenyl or C₇₋₁₀phenyl-alkyl,
R₁₂ is hydrogen, C₁₋₃alkyl or a group of formula -CH(R₁₃)-X₁,
R₁₃ is CH₂OH, -(CH₂)₂-OH, -(CH₂)₃-OH, or -CH(CH₃)OH or represents the substituent attached to the α-carbon atom of a natural or synthetic α-amino acid (including hydrogen),
X₁ is a group of formula -COOR₇, -CH₂OR₁₀ or 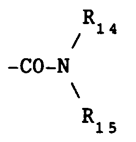
R₇ is hydrogen or C₁₋₃alkyl,
R₁₀ is hydrogen or the residue of a physiologically acceptable, physiologically hydrolysable ester,
R₁₄ is hydrogen or C₁₋₃alkyl and
R₁₅ is hydrogen, C₁₋₃alkyl, phenyl or C₇₋₁₀phenylalkyl, and
R₁₆ is hydrogen or hydroxy,
with the proviso that
when R₁₂ is -CH(R₁₃)-X₁ then R₁₁ is hydrogen or methyl,
wherein the residues B, D and E have the L-configuration, and the residues in the 2- and 7-position and any residues Y₁ 4) and Y₂ 4) each independently have the (L)- or (D)- configuration,
in free form or in pharmaceutically acceptable salt or complex thereof,
in the manufacture of a pharmaceutical composition for use in preventing or treating NSAID induced gastro-intestinal lesions or ulcers.

2. Use of a compound of formula I according to claim 1, in the manufacture of a pharmaceutical composition for use in reducing or suppressing the gastro-intestinal lesions and ulcers associated with a NSAID therapy.

3. Use according to claim 1 or 2, wherein the compound of formula I is selected from:

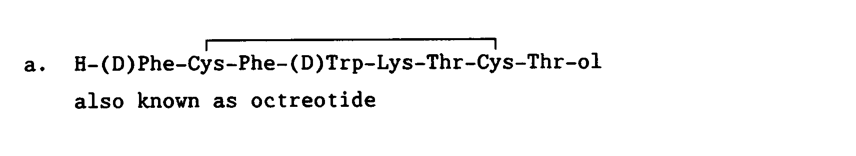

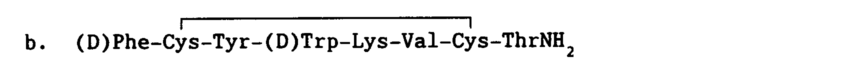

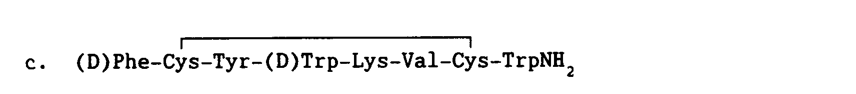

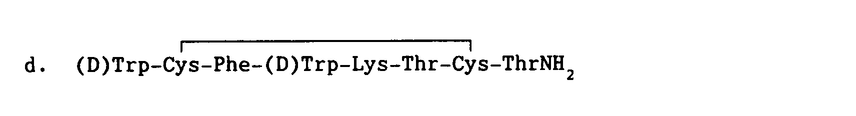

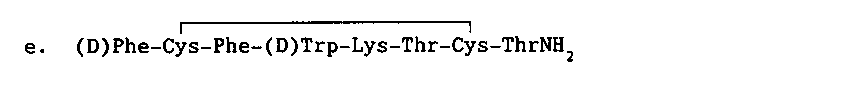

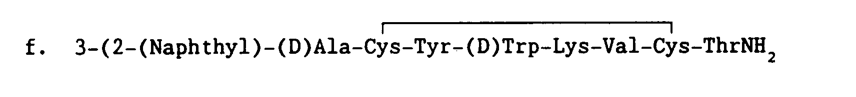

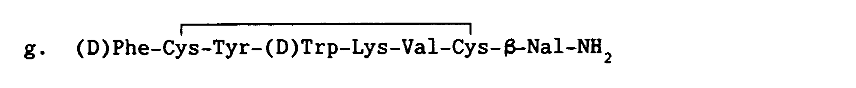

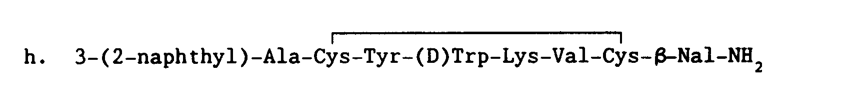

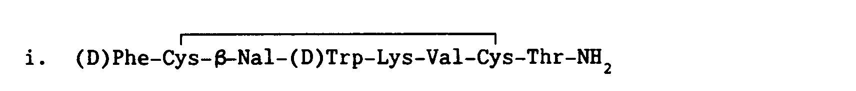

4. A kit or package combining a NSAID pharmaceutical composition with a pharmaceutical composition comprising a compound of formula I as defined in claim 1, in free form or in pharmaceutical acceptable salt or complex form.

5. A kit or package according to claim 4 wherein the compound of formula I is selected from compounds a. to i. as defined in claim 3.
